# EUROPEAN PATENT APPLICATION

(11) **EP 3 995 487 A1**
(43) Date of publication of application: **11.05.2022**
(21) Application number: 20206201.4
(22) Date of filing: 06.11.2020
(51) Int. Cl.: C07C 273/02, B01J 2/02, C07C 273/04, C07C 273/14, C05C 9/00

(54) **UREA PRILLING PROCESS**

(71) Applicant: CASALE SA, 6900 Lugano (CH)
(72) Inventor: MARRONE, Leonardo, 21020 Mercallo (VA) (IT); BERETTI, Andrea, 22069 Rovellasca (CO) (IT)
(74) Representative: M. Zardi & Co S.A.

(57) **Abstract**

A process of prilling of a urea melt comprising the use of calcium lignosulfonate or carboxymethyl starch as additive to obtain a formaldehyde-free urea product.

## Description

### Field of the invention

The present invention relates to a process for the prilling of urea.

### Prior Art

Urea is produced industrially by reacting ammonia and carbon dioxide at suitable urea-forming conditions, typically at a high pressure and high temperature.

Urea is synthesized at a synthesis pressure above 100 bar obtaining a reaction effluent containing urea, water and unconverted reagents mostly in the form of ammonium carbamate. Due to the equilibrium reached in the reaction environment, the amount of unconverted matter in the reaction effluent is significant and the reaction effluent is normally processed for its recover.

In the widely used stripping processes, the reaction effluent is heated in a high-pressure stripper, possibly in the presence of a stripping agent, to decompose the ammonium carbamate and extract gaseous ammonia and carbon dioxide. These are condensed in a high-pressure condenser and recycled to the synthesis reactor. When used, the stripping agent is generally gaseous carbon dioxide or gaseous ammonia.

Said high-pressure stripper and high-pressure condenser may operate at substantially the same pressure as the synthesis reactor, thus forming a high-pressure synthesis section or loop. The urea-containing effluent of the stripper is then processed in one or more recovery sections at a lower pressure to further recover unconverted reagents, obtaining a purified aqueous solution of urea. Said purified solution is essentially made of urea and water and may contain for example about 65% to 70% urea by weight, the balance being water and unavoidable impurities.

Many applications require urea in a solid form. The production of solid urea is also termed finishing or product-shaping.

An overview of the techniques for the urea synthesis and the subsequent product shaping can be found in the relevant literature, for example in the Ullmann's Encyclopedia of industrial chemistry.

The prilling process is one of the two most common techniques for urea shaping, the other being granulation. The solid particles obtained by the prilling process are named prills whilst the particles obtained by the granulation process are termed granules.

For use in a prilling process, the aqueous solution is first converted into a urea melt by removing water, e.g. in a suitable evaporation section; the so obtained urea melt is distributed in the the form of droplets in a prilling equipment where the urea droplets solidify as they fall down in the presence of a counter-current flow of cooling air.

The prilling equipment normally takes the form of a prilling tower. The urea melt may contain more than 98% or more than 99% of urea. A purity of 99.5% or even higher may be required. This is in contrast with granulation where a higher content of water, such as 3% or 4%, can be generally accepted in the source urea.

The droplets may be produced with showerheads or with a suitable prilling bucket installed on top of the prilling tower. The prilling bucket is a rotating bucket with a perforated side surface fed with the urea melt, wherein the rapid rotation of the bucket generates the droplets. A prilling process in a tower with a rotating bucket is described, for example, in WO 2004/101131. In the prilling process, the droplets solidify whilst falling downward through the tower without any further addition of urea. The solid particles are collected at the base of the prilling tower.

An important aspect of the product shaping techniques is the mechanical properties of the so obtained product, particularly the crushing strength. The prilling process is generally considered inferior to the granulation process in terms of the crushing strength of the product; however prilling is still widely used and many prilling towers are in operation, so that there is an interest to improve the quality of the prilled product.

It is known that the mechanical properties of the prills can be improved by a suitable additive. To this purpose, a known and widely used additive is formaldehyde which may be added as such or in the form of a formaldehyde-containing solution. The addition of formaldehyde however introduces relevant concerns of health and environmental sustainability. Any formaldehyde added before the prilling process will inevitably contaminate the urea product. Particularly for certain applications, e.g. the production of feed grade urea, formaldehyde is not desired.

The term feed grade urea denotes urea suitable to be used directly as a feed component for cattle, e.g. ruminants. Feed grade urea is often produced with the prilling technique, because the typical size of the prills is suitable for this use.

Another field where formaldehyde is not desired is, for example, the production of DEF (diesel exhaust fluid) grade urea. This term denotes urea for use in the selective catalytic removal of NOx from flue gas. Urea for this use must meet stringent quality requirements, for example compliance with the DIN 70070 norm.

There is therefore an effort to find an additive suitable to replace formaldehyde in a prilling process for production of formaldehyde-free urea. Any such additive must be safe and economically acceptable. Also, the additive should be effective at low concentrations to maintain the desired properties of the urea product, for example nutritional value of feed-grade urea for cattle.

### Summary of the invention

The invention aims to solve the above mentioned problem. In particular, the invention aims to find a safe and economically acceptable additive for a urea prilling process, which can increase the mechanical properties of the urea prills and therefore can replace formaldehyde.

The invention is based on the finding that calcium lignosulfonate and carboxymethyl starch can be used for the above purpose. These additives are fully safe, they do not pose health concerns, and their cost is similar to that of the common formaldehyde-containing solutions like UF80.

Accordingly, the aims are reached with a process for the prilling of a urea melt according to claim 1.

The applicant has found experimentally that the above additives provide a crushing strength of the urea prills fully comparable to that obtainable with the addition of formaldehyde. This result can be achieved with a total amount of additive not greater than 1.0%, which means the additives of the invention are also effective at low concentration and can be used without significant alteration of the urea product. Said total amount may include only one of the above mentioned additives or both. The additives may be used together in a mixture.

Particularly, a feed grade urea product can be obtained with the desired properties, including an acceptable content of nitrogen. In most cases a minimum content of nitrogen of 46% by weight is required in the feed grade urea and the formaldehyde-free urea produced with the process of the invention is able to satisfy this requirement.

With regard to the cost issues, the additives of the present invention may introduce an additional cost of about 4.5 to 5.5 EUR per metric ton of urea at the current market conditions. The conventional addition of UF80 at a concentration of 0.5% introduces a cost of around 4.6 EUR per metric ton. Therefore the additives of the invention are also economically viable.

### Preferred embodiments

The total amount of additive in the urea melt is preferably 0.1% to 1.0% weight on dry basis relative to the urea. More preferably the amount is 0.3% to 0.8% weight on dry basis relative to the urea.

The additives of the invention may be used singularly or in combination.

In embodiments using only one additive, said total amount of additive is the amount of the selected additive, that is carboxymethyl starch or calcium lignosulfonate.

In embodiments using both additives, said total amount of additive is the amount of carboxymethyl starch plus the amount of calcium lignosulfonate.

In embodiments using both additives, the amount by weight of carboxymethyl starch and the amount by weight of calcium lignosulfonate are preferably the same, i.e. the ratio is 1:1.

In embodiments using both additives, they can be added separately or together as a mixture.

When the prilling additive includes calcium lignosulfonate, a particularly preferred amount of calcium lignosulfonate in the urea melt is 0.7% or about 0.7% weight on dry basis relative to the urea. For example the amount may be 0.65% to 0.75%.

When the prilling additive includes carboxymethyl starch a particularly preferred amount of carboxymethyl starch in the urea melt is 0.5% or about 0.5% weight on dry basis relative to the urea. For example the amount may be 0.45% to 0.55%.

The term urea melt denotes a highly concentrated solution which is typically obtained after evaporation of water from an aqueous solution of urea. The urea melt may contain more than 98% weight of urea and preferably more than 99% weight of urea. More preferably the urea melt contains more than 99.5% urea, for example 99.6% or 99.7% urea. This is because water contained in the source urea is detrimental to the prilling process and may affect the strength of the prills obtainable with the process.

In the prilling process, the urea melt may be converted into droplets by means of one or more showerheads or by means of one or more rotating prilling bucket. A showerhead or a prilling bucket may be installed on top of a prilling tower.

The urea prills obtained with the process of the invention may have an average diameter not greater than 2.0 mm, preferably not greater than 1.0 mm.

In a preferred application of the invention, feed grade urea prills are produced. A particularly preferred average diameter of feed-grade prills is 0.5 mm or about 0.5 mm.

The urea melt may be produced in a urea synthesis plant according to one of the known processes for the industrial synthesis of urea. These processes include for example the known CO2 stripping process introduced by Stamicarbon and the ammonia-stripping or self-stripping process introduced by Snamprogetti.

In cases of practical interest, urea is synthesized from ammonia and carbon dioxide at a synthesis pressure obtaining a reaction effluent containing urea, water and unconverted ammonium carbamate; said reaction effluent is processed in one or more recovery sections at a lower pressure to recover unconverted reagents, obtaining a purified aqueous solution of urea; said aqueous solution is then processed with an evaporation step in a suitable evaporation section to remove water and obtain the urea melt.

The prilling additives of the present invention can be added before or after the evaporation step. That is to say, each additive (calcium lignosulfonate or carboxymethyl starch), or a mixture thereof, can be added to the purified aqueous solution of urea before evaporation or can be added to the urea melt obtained after evaporation. The additive or mixture can be added directly to a stream of urea solution or melt, or can be added in a suitable tank.

In accordance with the above, a preferred application of the invention is a process for the production of solid urea comprising:
reaction of ammonia and carbon dioxide at a synthesis pressure obtaining a reaction effluent containing urea, water and unconverted ammonium carbamate; processing said reaction effluent at a lower pressure to recover unconverted reagents, obtaining a purified aqueous solution of urea; subjecting said aqueous solution of urea to an evaporation process to remove water and obtain the urea melt, the process further comprising:
adding at least one of calcium lignosulfonate and carboxymethyl starch, as a prilling additive, to the purified aqueous solution or to the urea melt;
wherein no formaldehyde is added to the purified aqueous solution or to the urea melt, so that the urea melt contains no added formaldehyde;
converting the urea melt with the additive into a solid product by a prilling process wherein the prilling process includes distributing the urea melt in the form of droplets in a prilling tower wherein the droplets fall down the tower and solidify in the presence of a counter-current upflowing cooling air.

### Description of the figure(s)

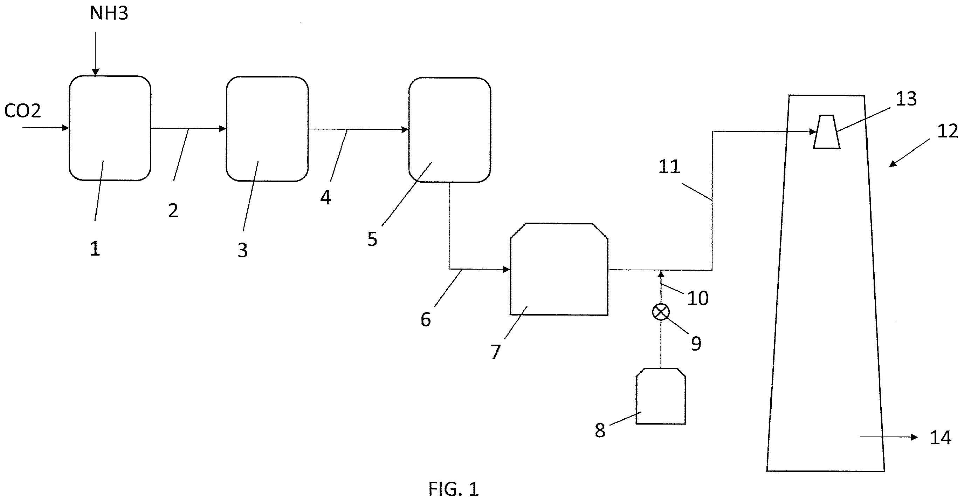

Fig. 1 illustrates a scheme of an implementation of the invention. The following items and process streams are shown.
Item 1 is a synthesis section where ammonia (NH3) and carbon dioxide (CO2) are reacted at high temperature and high pressure to form urea. Said synthesis section 1 may include at least a reactor, a high-pressure stripper and a high-pressure condenser.
Stream 2 is an aqueous solution containing urea, water and unconverted ammonium carbamate. This solution can be withdrawn from the stripper of the synthesis section 1.
Item 3 is a recovery section where the unconverted reagents contained in the solution 2 are recovered and recycled back to the section 1. The unconverted reagents are normally recovered by one or more steps of heating the urea-containing solution for decomposition of ammonium carbamate into gaseous ammonia and CO2, and condensation of said reagents into a carbamate-containing solution which can be pumped back to the reactor or to the condenser of the section 1. The recovery section 3 may operate at one or more pressure levels.
Stream 4 is a purified solution obtained from the recovery section 3. This purified solution 4 contains urea, water and unavoidable impurities.
Item 5 is an evaporation section which removes water from the solution 4.
Stream 6 is a urea melt.
Item 7 is a urea melt tank.
Item 8 is an additive tank.
Item 9 is an additive metering device.
Stream 10 denotes the additive (calcium lignosulfonate and/or carboxymethyl starch) added to the urea melt from the tank 7.
Stream 11 is urea melt containing the additive.
Item 12 is a prilling tower.
Item 13 is a prilling bucket installed in the prilling tower 12.
Stream 14 is the solid product (urea prills) collected at the bottom of the prilling tower 12.

In alternative embodiments, the additive 10 may be added directly in the melt tank 7 or it may be added to the urea solution 4 before evaporation.

The additive 10 may be a mixture of calcium lignosulfonate and carboxymethyl starch. In some embodiments, calcium lignosulfonate and carboxymethyl starch may be added separately to the urea solution 4 and/or to the urea melt 6. For each additive a respective tank and a respective metering device can be provided. When added separately, the calcium lignosulfonate and carboxymethyl starch may be added at the same or a different location. The total amount of the two additives is preferably in the range 0.1% to 1.0% weight on dry basis relative to the urea.

## Claims

1. A process for the prilling of a urea melt wherein:
the prilling process includes distributing urea melt in the form of droplets in a prilling tower and urea droplets solidify as they fall down the tower in counter-current with upflowing air;
the urea melt contains at least one of calcium lignosulfonate and carboxymethyl starch as a prilling additive, and
the urea melt contains no added formaldehyde.

2. A process according to claim 1, wherein the total amount of said prilling additive in the urea melt is 0.1% to 1.0% weight on dry basis relative to the urea.

3. A process according to claim 1 wherein the total amount of said prilling additive in the urea melt is 0.3% to 0.7% weight on dry basis relative to the urea.

4. A process according to any of the previous claims wherein the amount of calcium lignosulfonate in the urea melt is 0.7% weight on dry basis relative to the urea.

5. A process according to any of the previous claims wherein the amount of carboxymethyl starch in the urea melt is 0.5% weight on dry basis relative to the urea.

6. A process according to any of the previous claims wherein the urea melt contains, as a prilling additive, calcium lignosulfonate and carboxymethyl starch, preferably in a ratio of 1:1 by weight.

7. A process according to any of the previous claims wherein the urea melt contains more than 98% weight of urea and preferably more than 99% weight of urea.

8. A process according to any of the previous claims wherein the urea melt is converted into droplets by means of one or more showerheads or by means of one or more rotating prilling bucket.

9. A process according to any of the previous claims wherein the so obtained urea prills have an average diameter not greater than 2.0 mm, preferably bit greater than 1.0 mm.

10. A process according to any of the previous claims wherein the so obtained urea prills are feed grade.

11. A process according to any of the previous claims wherein the urea melt is produced in a urea synthesis plant wherein urea is synthesized from ammonia and carbon dioxide at a synthesis pressure obtaining a reaction effluent containing urea, water and unconverted ammonium carbamate; said reaction effluent is processed in one or more recovery sections at a lower pressure to recover unconverted reagents, obtaining a purified aqueous solution of urea; said aqueous solution being processed in an evaporation section to remove water and obtain the urea melt, wherein:
said prilling additive is added to the purified aqueous solution before evaporation or is added to the urea melt.

12. A process for the production of solid urea comprising:
reaction of ammonia and carbon dioxide at a synthesis pressure obtaining a reaction effluent containing urea, water and unconverted ammonium carbamate; processing said reaction effluent at a lower pressure to recover unconverted reagents, obtaining a purified aqueous solution of urea; subjecting said aqueous solution of urea to an evaporation process to remove water and obtain the urea melt, the process further comprising:
adding at least one of calcium lignosulfonate and carboxymethyl starch, as a prilling additive, to the purified urea aqueous solution or to the urea melt;
wherein no formaldehyde is added to the purified aqueous solution or to the urea melt, so that the urea melt contains no added formaldehyde;
converting the urea melt with the additive into a solid product by a prilling process wherein the prilling process includes distributing the urea melt in the form of droplets in a prilling tower wherein the droplets fall down the tower and solidify in the presence of a counter-current upflowing cooling air.

13. A process according to claim 12 wherein the prilling additive is added to the aqueous solution or to the urea melt in an amount which is 0.1% to 1.0% weight on dry basis relative to the urea, preferably 0.3% to 0.7%.

14. A process according to claim 12 or 13, wherein both calcium lignosulfonate and carboxymethyl starch are used as prilling additives, and they are added separately or as a mixture to the purified aqueous solution before evaporation or to the urea melt.

15. A process according to any of claims 12 to 14 wherein the urea is feed grade.
